(19) Europäisches Patentamt
European Patent Office
Office européen des brevets

(11) Publication number: **0 041 359**
**B1**

(12) # EUROPEAN PATENT SPECIFICATION

(45) Date of publication of patent specification: **14.11.84**

(21) Application number: **81302336.3**

(22) Date of filing: **27.05.81**

(51) Int. Cl.[3]: **C 07 D 233/64,**
C 07 D 233/68,
C 07 D 487/04, A 61 K 31/415

(54) **Process for the preparation of heterocyclylalkyl guanidines; intermediates and their preparation.**

(30) Priority: **31.05.80 GB 8017881**

(43) Date of publication of application:
**09.12.81 Bulletin 81/49**

(45) Publication of the grant of the patent:
**14.11.84 Bulletin 84/46**

(84) Designated Contracting States:
**AT BE CH DE FR GB IT LI LU NL SE**

(56) References cited:
**FR-A-2 254 311**
**US-A-3 763 179**

**Organic Chemistry, N.L. Allinger et al., Worth Publishers 1971, pp. 169-170 and 388-389**

(73) Proprietor: **SMITH KLINE & FRENCH LABORATORIES LIMITED**
**Mundells**
**Welwyn Garden City Hertfordshire, AL7 1EY (GB)**

(72) Inventor: **Hills, Derek William**
**63 Daniells**
**Welwyn Garden City Hertfordshire AL7 1QT (GB)**
Inventor: **White, George Raymond**
**16 Ashley Gardens**
**Harpenden Hertfordshire (GB)**

(74) Representative: **Johnston, Walter Paul**
**P.O. Box 39**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 359 B1

## Description

This invention relates to a process for preparing certain heterocyclylalkylguanidines, to intermediates for use in this process and to a process for preparing these intermediates.

In recent years it has been recognised that certain heterocyclylalkylguanidines possess useful biological activity. For example British Patent Specification No. 1,531,237 discloses and claims compounds of formula (I):—

$$Het—CH_2SCH_2CH_2NH—C(=NH)—NH(CH_2)_3—Het' \quad (I)$$

where Het is a 4-imidazolyl, 5-methyl-4-imidazolyl, 5-ethyl-4-imidazolyl, 5-halo-4-imidazolyl, 2-thiazolyl, 3-isothiazolyl, 4-halo-3-isothiazolyl, 2-pyridyl, 3-halo-2-pyridyl, 3-hydroxy-2-pyridyl, 3-methoxy-2-pyridyl or 3-ethoxy-2-pyridyl group and Het′ is a 4-imidazolyl group; and hydrates, pharmaceutically acceptable salts and hydrated pharmaceutically acceptable salts thereof.

Compounds of formula (I) have been prepared either by reacting a compound of formula (II) or (III):—

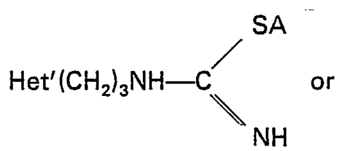

or

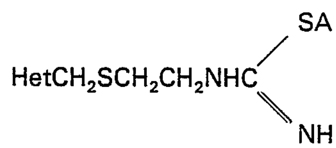

(II) (III)

with a complementary amine of formula (IV) or (V):—

$HetCH_2SCH_2CH_2NH_2$ or $Het'(CH_2)_3NH_2$

(IV) (V)

Alternatively compounds of formula (I) were prepared by reacting a compound of formula (VI) or (VII):—

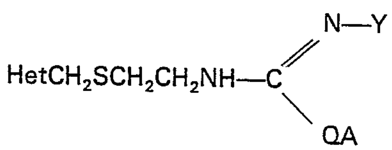

or

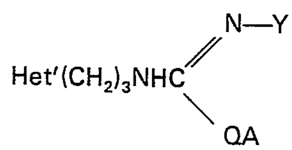

(VI) (VII)

with a complementary amine of formula (IV) or (V) above to form a compound of formula (VIII):—

$$HetCH_2SCH_2CH_2NH—C(=NY)—NH(CH_2)_3Het' \quad (VIII)$$

which is then converted into a compound of formula (I). In formulae (II) to (VIII) Het and Het′ are as defined with reference to formula (I); A is alkyl, Q is oxygen or sulphur and Y is a group such that the compounds of formula (VIII) may be converted easily into the corresponding compound of formula (I), for example Y may be benzoyl or cyano.

We have now invented a process for preparing heterocyclylalkyl guanidines including those of formula (I) above with an improved degree of purity in particular the formation of bis compounds for example of formula (Ia):—

$$HetCH_2SCH_2CH_2NH_2—C(=NH)—NHCH_2CH_2SCH_2Het \quad (Ia)$$

as biproducts is avoided.

Accordingly the present invention provides a process for preparing compounds of formula (IX):—

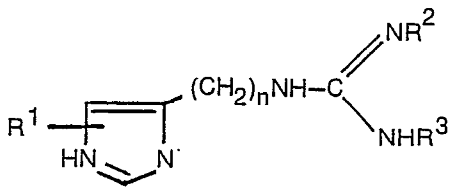

(IX)

where $R^1$ is attached to a carbon atom in the imidazole moiety and is hydrogen, halogen or alkyl; $R^2$ is hydrogen, or a group convertible into hydrogen under acid conditions; $R^3$ is an optionally substituted alkyl group and *n* is 2, 3 or 4; which comprises reacting a compound of formula (X):—

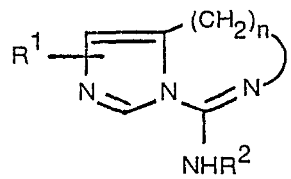

(X)

where *n* and $R^1$ are as defined with reference to formula (IX) and $R^2$ is a group convertible into hydrogen under acid conditions, with a compound of formula (XI):—

$$R^3NH_2 \qquad (XI)$$

where $R^3$ is as defined with reference to formula (IX) in the presence of a polar organic solvent and thereafter optionally converting in the compound so obtained the group $R^2$ into hydrogen under acid conditions.

One particularly important compound which can be made by the process of this invention is N - [3 - (4 - imidazolyl)propyl] - N′ - [2 - (5 - methyl - 4 - imidazolylmethylthio)ethyl]guanidine (impromidine) which is useful as a histamine $H_2$-agonist.

The nature and identity of $R^1$ are not critical to the success of the process, the group being selected having regard to the particular guanidine to be prepared.

Examples of halogens which $R^1$ represents are chlorine and bromine.

Examples of alkyl groups which $R^1$ represents are $C_{1-4}$ alkyl groups, particularly methyl.

Preferable $R^1$ occupies position 5 of the imidazolyl moiety.

In particular $R^1$ is hydrogen.

By way of example $R^2$ is benzoyl or cyano but preferably it is benzoyl.

Using the process of this invention it is possible to introduce a wide variety of groups $R^3$. Thus the group $R^3$ may be selected on the basis of the properties which it is desired that the product should possess.

$R^3$ may represent an alkyl group for example a $C_{1-4}$ alkyl group and particularly a methyl, ethyl or n-propyl group; or a phenyl substituted alkyl group, for example benzyl.

$R^3$ may also represent a substituted alkyl group of formula (XII):—

$$Het^2(CH_2)_pZ(CH_2)_q— \qquad (XII)$$

where $Het^2$ is a 2-furanyl or 2-thienyl group optionally substituted in the 5-position with a group $R^5R^6N(CH_2)_r$— where $R^5$ and $R^6$ are $C_{1-4}$ alkyl or together with the nitrogen atom to which they are attached form a pyrrolidino, piperidino or morpholino group and r is 1 to 4; a 4-imidazolyl group optionally substituted in the 5-position with $C_{1-4}$ alkyl or halogen; a 2-pyridyl group optionally substituted in the 3-position with $C_{1-4}$ alkyl, $C_{1-4}$ alkoxy, halogen, amino or hydroxy; a 2-thiazolyl group or a 2-guanidino-4-thiazolyl group; Z is sulphur or methylene; when Z is sulphur *p* is 1 or 2 and *q* is 2 or 3 provided that $p + q$ is 3 or 4 and when Z is methylene *p* is 0, 1 or 2 *q* is 2 or 3 and $p + q$ is 2, 3 or 4.

With reference to formula (XII) examples of $C_{1-4}$ alkyl groups which $R^5$ and $R^6$ represent are methyl, ethyl, and *n*-propyl. Preferably $R^5$ and $R^6$ are methyl, particularly where r is 1.

When $Het^2$ is a 4-imidazolyl group substituted in the 5-position with $C_{1-4}$ alkyl or halogen preferably the substituents are methyl or bromine.

When $Het^2$ is a 2-pyridyl group substituted in the 3-position with $C_{1-4}$ alkyl, $C_{1-4}$ alkoxy, or halogen preferably the substituent is methyl, methoxy or bromine.

Preferably *p* is 1 and *q* is 2.

Preferably Z is sulphur.

Particular meanings of $R^3$ in formula (XII) are:—

2-(2-guanidino-4-thiazolylmethylthio)erthyl-;
3-(4-imidazolyl)-propyl-;
4-(4-imidazolyl)butyl-;
4-(5-methyl-4-imidazolyl)butyl-;
2-(5-methyl-4-imidazolylmethylthio)ethyl;
2-(5-dimethylaminomethyl-2-furanylmethylthio)ethyl; and
2-(5-dimethylaminomethyl-2-thienylmethylthio)ethyl;

of which 2-(5-methyl-4-imidazolylmethylthio)ethyl is important because it is present in the compound impromidine.

$R^3$ may also represent a substituted alkyl group of formula (XIII):—

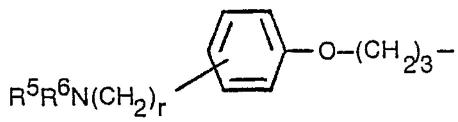

(XIII)

where the group $R^5R^6N(CH_2)_r$— is as defined with reference to formula (XII) and occupies the 3- or 4-position of the phenyl group.

Examples and preferred values of $R^5$, $R^6$ and *r* are as discussed above with reference to formula (XII).

A particular meaning of $R^3$ having formula (XIII) is:—

3-(3-dimethylaminomethylphenoxy)propyl

The reaction is carried out in a polar organic solvent, the choice of which is not critical to the success of the reaction provided that it is substantially inert to the reagents and product. Examples of suitable solvents are N,N-dimethylformamide and $C_{1-4}$ alkanols, particularly ethanol.

The reaction is generally carried out at moderate temperatures for example from room temperature to the reflux temperature of the solvent.

The period required for the reaction to proceed to completion depends upon the reaction condition. The reaction can be followed by standard techniques for example thin layer chromatography and is stopped when it is indicated that the reaction is substantially completed.

The product can then be isolated from the reaction mixture by conventional methods.

Compounds of formula (IX) where $R^2$ is a group convertible into hydrogen under acid condition, for example cyano or benzoyl, can be converted into the corresponding compounds of formula (IX) where $R^2$ is hydrogen by acid hydrolysis using for example a mineral acid in particular hydrochloric or sulphuric acid.

Compounds of formula (IX) above where *n* is 2 or 3 and $R^3$ is a group of formula (XII) or (XIII) are predominantly histamine $H_2$-agonists. Compounds of formula (IX) above where *n* is 4 and $R^3$ is a group formula (XII) or (XIII) are predominantly histamine $H_2$-antagonists.

Compounds of formula (IX) above where $R^3$ is a group of formula (XII) where Z, p and q are as previously defined; $Het^2$ is 2-guanidino-4-thiazolyl; *n* is 2, 3 or 4, $R^2$ is hydrogen, cyano or benzoyl and $R^1$ is hydrogen, $C_{1-4}$ alkyl or halogen provided that *n* is 4 when $R^1$ and $R^2$ are hydrogen are described as histamine $H_2$-antagonists in European Patent Specification No. 0 010 418.

Compounds of formula (IX) above where $R^1$ is in position 5 and is hydrogen, $C_{1-4}$ alkyl or halogen $R^3$ is a gorup of formula (XII) where Z, p and q are as previously defined; $Het^2$ is 2-furanyl or 2-thienyl substituted with a group $R^5R^6N(CH_2)_r$ as previously defined; $R^2$ is hydrogen or cyano, *n* is 2, 3 or 4 and compounds of formula (IX) where $R^3$ is a group of formula (XIII) and *n* is 2, 3 or 4 are described as histamine $H_2$-antagonists in European Patent Specification No. 0 002 930.

The intermediates of formula (X) are novel and form a further aspect of the invention.

Specific intermediates of formula (X) are:—

8-benzamido-4,5-dihydroimidazo(3,4-c)-1,3-diazepine and
9-benzamido-4,5,6,7-tetrahydroimidazo(3,4-c)-1,3-diazocine.

The intermediates (X) can be prepared by a process which comprises cyclising in a polar organic solvent a compound of formula (XIV):—

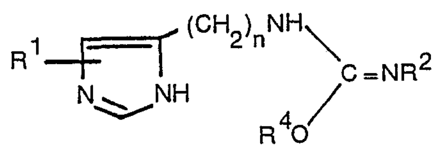

(XIV)

where $R^1$, $R^2$ and *n* are as defined with reference to formula (X) and $R^4$ is an optionally substituted aryl group where necessary in the presence of a non-nucleophilic base.

Preferably $R^2$ in the compound of formula (XIV) is benzoyl.

Examples of groups which $R^4$ represents are phenyl optionally substituted with one or more $C_{1-4}$ alkyl, $C_{1-4}$ alkoxy, halogen or nitro substituents.

Preferably $R^4$ is phenyl.

Examples of non-nucleophilic bases which can be used in this process are alkali metal and alkaline earth metal hydrides for example sodium and calcium hydride, alkali metal t-butoxides for example potassium t-butoxide and bicyclic diazalkenes for example 1,5-diazabicyclo(4.3.0)non-5-ene and 1,5-diazabicyclo(5.4.0)undec-5-ene.

Preferably the base is sodium hydride.

When $R^2$ is cyano and *n* is 2 or 3 the base can be omitted. The reaction is carried out in a non-interfering polar organic liquid diluent or solvent the choice of which is not critical to the success of the reaction provided that it is substantially inert to the reagents and product. Examples of such solvents include N,N-dimethylformamide and dimethylsulphoxide. When the base is a hydride the reaction medium must be dry.

The reaction is generally carried out at low temperatures for example 0°C to room temperature, 5—10°C being preferred.

The time required for the reaction to go to completion depends upon the reagents employed and the precise reaction conditions. In any particular case the process can be monitored by a conventional technique for example thin layer chromatography and the reaction mixture worked up by conventional methods.

The intermediates of formula (XIV) can in turn be prepared by reacting an amine of formula (XV):—

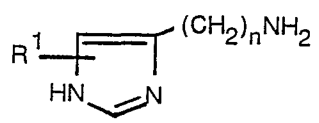

(XV)

where $R^1$ and *n* are as defined with reference to formula (XIV); with a compound of formula (XVI):—

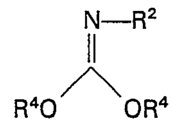

(XVI)

where $R^2$ and $R^4$ are as defined with reference to formula (XIV).

This reaction is carried out in a polar organic solvent, for example a halogenated hydro carbon, in particular methylene chloride and a $C_{1-4}$ alkanol, in particular ethanol at moderate to low temperature for example, less than 10°C and preferably between 5° and 7°C.

The intermediates (XIV) particularly where $R^1$ and $R^4$ are as previously defined, $R^2$ is CN and *n* is 2 or 3 can cyclize *in situ*. Thus intermediates of formula (X) where $R^1$ is as previously defined, $R^2$ is CN and *n* is 2 or 3 can be prepared by reacting an amine of formula (XV) where $R^1$ as previously defined and *n* is 2 or 3 with a compound of formula (XVI) where $R^2$ is cyano and $R^4$ is as previously defined to form a compound of formula (XIV) where $R^1$ and $R^4$ are as previously defined, $R^2$ is CN, and *n* is 2 or 3 which is allowed to cyclise *in situ*.

The following Examples illustrate the invention.

## EXAMPLES

### Example 1

(i) A solution of 4-methylhistamine was made *in situ* by adding the dihydrochloride (9.9 g) to a solution of sodium ethoxide in ethanol (from 2.3 g sodium and 50 ml ethanol) heating the mixture to reflux with stirring, cooling the mixture in ice and filtering the cooled solution to remove precipitated sodium chloride.

The 4-methylhistamine solution was added dropwise with stirring over 15 min to a cooled (5°C) solution of diphenyl cyanoiminocarbonate (11.9 g) in methylene dichloride keeping the temperature

between 5—7°C throughout the addition. When the addition was completed the reaction mixture was allowed to warm to room temperature with stirring over 30 min.

The mixture was extracted with sodium bicarbonate solution (5% w/v), the organic phase was dried over magnesium sulphate and the solvent evaporated yielding an oil which solidified on trituration with diethyl ether. The solid was dissolved in boiling ethanol and precipitated with n-hexane yielding 7-cyanoamino-3,4-dihydroimidazo(3,4-c)-1,3-pyrimidine (4.0 g) m.p. 249—250°C.

(ii) A mixture of 2-(5-methyl-4-imidazolylmethylthio)-ethylamine (2.5 g) and 7-cyanoamino-3,4-dihydroimidazo-(3,4-c)-1,3-pyrimidine (2.23 g) in ethanol (50 ml) were heated under reflux for 20 hr. The ethanol was evaporated leaving a oily residue which on addition of water formed a white crystalline solid which was recrystallised from water yielding N - cyano - N′ - [2 - (5 - methyl - 4 - imidazolyl)ethyl] - N″ - [2 - (5 - methyl - 4 - imidazolylmethylthio)ethyl]guanidine (2.10 g) m.p. 190—191°C.

Example 2

(i) A solution of 3-(4-imidazolyl)propylamine in ethanol was prepared *in situ* from the dihydrochloride (4.95 g) by the method of Example 1 (i) with a solution of sodium ethoxide (from 1.15 g sodium and 50 ml ethanol) in ethanol.

The 3-(4-imidazolyl)propylamine solution was added dropwise with stirring over 15 min to a cooled (5°C) solution of diphenyl benzoyliminocarbonate (7.93 g) in dichloromethane (75 ml), keeping the reaction temperature between 5—7°C throughout the addition. When the addition was complete the mixture was allowed to warm to room temperature over 30 min with stirring.

The volume of the mixture was evaporated to 25 ml poured into water (250 ml) and chilled for *ca* 16 hr. The white solid which deposited was recrystallised from aqueous ethanol yielding N-benzoyl-N-[3-(4-imidazolyl)propyl]-O-phenyl isourea (6.06 g) as a white crystalline solid.

(ii) N-benzoyl-N′-(3-(4-imidazolyl)propyl)-O-phenyl isourea (5.0 g) was added to a stirred suspension of sodium hydride (0.5 g NaH, weighed as a 50% dispersion in oil and washed before use with b.p. 40—60° petroleum ether) in dry dimethylformamide (50 ml). The addition was carried out over 10 min with cooling, keeping the temperature between 5 and 10°C. The reaction mixture was stirred for a further 10 minutes and then allowed to warm to room temperature. Stirring was continued until T.L.C. ($SiO_2$ plate $F_{254}$; eluted with EtOAc:MeOH:$NH_4OH$ s.g. 0880 (5:1:1) indicated that the reaction was complete. The reaction mixture was poured into water, and allowed to stand over-night. The precipitated product was filtered, washed and recrystallised by dissolving in ethanol (30 ml) filtering through diatomaceous earth diluting the filtrate with an equal volume of water, and allowing the diluted filtrate to stand overnight at 10°C. The product was isolated by filtration and dried yielding 8-benzamido-4,5-dihydroimidazo(3,4-c)-1,3-diazepine (2.31 g) m.p. 158—160°C.

Example 3

(i) A solution of 4-(4-imidazolyl)butylamine (1.39 g) in ethanol (25 ml) was added dropwise with stirring over 10 min to a solution of diphenyl benzoylimino carbonate (3.17 g) in dichloromethane (25 ml) with cooling to maintain the temperature at between 5—6°C. When the addition was completed the reaction mixture was stirred for a further 30 min without cooling. The volume of the reaction mixture was reduced to 10 ml by evaporation and the residue was washed with water by decantation yielding N-benzoyl-N-[4-(4-imidazolyl)butyl]-O-phenyl isourea which was used without purification in the next stage.

(ii) A solution of N-benzoyl-N′-[4-(4-imidazolyl)butyl]-O-phenyl isourea (2.51 g) in dimethylformamide (15 ml) was added dropwise with stirring over 10 min to a suspension of sodium hydride (0.5 g; weighed as a 50% dispersion in oil) in dimethylformamide (10 ml), keeping the temperature at between 5—7°C throughout the addition. The mixture was then stirred for 30 min at room temperature and poured into water (200 ml). An oil deposited which solidified. The solid was filtered and recrystallised from ethanol/diethyl ether giving 9-benzamido-4,5,6,7-tetrahydroimidazo(3,4-c)-diazocine (1.05 g) m.p. 102—103°C.

Example 4

A solution of 3-(4-imidazolyl)propylamine in ethanol (400 ml) was made *in situ* from the dihydrochloride (4.0 g) and sodium ethoxide (from 0.92 g sodium and 400 ml ethanol) by the method of Example 1 (i). This solution was added dropwise with stirring over 15 min to a solution of diphenyl cyanoiminocarbonate (4.76 g) in dichloromethane (40 ml) keeping the temperature between 5—7°C throughout the addition.

When the addition was complete the reaction mixture was allowed to warm to room temperature and to stir for a further hour. Dichloromethane was evaporated leaving an ethanolic residue to which water was added causing an oil to deposit. The oil was washed with water by decantation and chilled. The oil solidified giving 8-cyanoamino-4,5-dihydroimidazo(3,4-c)diazepine m.p. 210—215°C.

Example 5

A solution of 2-(5-methyl-4-imidazolylmethylthio)ethylamine in ethanol (50 ml) was prepared

from the dihydrochloride (2.53 g) and sodium ethoxide in ethanol (from sodium 0.51 g and ethanol 50 ml) as described in Example 1(i). To this was added 8-benzamido-4,5-dihydroimidazo(3,4-c)-diazepine (2.54 g). The mixture so obtained was refluxed for 20 hr cooled, filtered and the solution evaporated leaving a residue which was triturated with water yielding N - benzoyl - N′ - [3 - (4 - imidazolyl)propyl] - N″ - [2 - (5 - methyl - 4 - imidazolylmethylthio)ethyl]guanidine as a white solid m.p. 98.5—100°C.

Example 6

(i) A mixture of 8-benzamido-4,5-dihydroimidazo(3,4-c)diazepine (2.54 g) and 4-(4-imidazolyl)-butylamine in ethanol (25 ml) were refluxed for 5 hr. The ethanol was evaporated and the residue was triturated with water, dried and triturated with diethyl ether. The residue solidified yielding crude N - benzoyl - N′ - [3 - (4 - imidazolyl)propyl] - N″ - [4 - (4 - imidazolyl)butyl]guanidine which was used in the next step without purification.

(ii) N - Benzoyl - N′ - [3 - (4 - imidazolyl)propyl] - N″ - [4 - (4 - imidazolyl)butyl]guanidine (3.9 g) was refluxed overnight with conc. hydrochloric acid (50 ml). The excess of hydrochloric acid was evaporated yielding a residue containing crude N - [3 - (4 - imidazolyl)propyl] - N′ - [4 - (4 - imidazolyl)butyl]guanidine trihydrochloride which was characterised as the tripicrate as follows. The residue was diluted with water and extracted with diethylether to remove benzoic acid. The aqueous phase was separated and an excess of aqueous sodium picrate solution was added to it. This deposited a yellow solid which was recrystallised from aqueous acetone yielding N - [3 - (4 - imidazolyl)propyl] - N′ - [4 - (4 - imidazolyl)butyl]guanidine tripicrate m.p. 206—208°C.

Example 7

Reaction of 2-(5-dimethylaminomethyl-2-furanylmethylthio)ethyl amine with 8 cyanoamino-4,5-dihydroimidazo(3,4-c)diazepine prepared as described in Example 4 yields N - cyano - N′[3 - (4 - imidazolyl)propyl] - N″ - [2 - (5 - dimethylaminomethyl - 2 - furanylmethylthio)ethyl guanidine. This can be hydrolysed with dilute hydrochloric acid to yield N - [3 - (4 - imidazolyl)propyl] - N′ - [2 - (5 - dimethylaminomethyl - 2 - furanylmethylthio)ethyl guanidine trihydrochloride which is characterised by conversion into the picrate. m.p. 144—146°C.

Example 8

Reaction of 2-(5-dimethylaminomethyl-2-thienylmethylthio)ethyl amine with 8-cyano-4,5-dihydroimidazo(3,4-c)diazepine prepared as described in Example 4 yields N - cyano - N′ - [3 - (4 - imidazolyl)propyl] - N″ - [2 - (5 - dimethylaminomethyl - 2 - thienylmethylthio)ethyl]guanidine. This can be hydrolysed with dilute hydrochloric acid to yield N - [3 - (4 - imidazolyl)propyl] - N′ - [2 - (5 - dimethylaminomethyl - 2 - thienylmethylthio)ethyl]guanidine trihydrochloride which is characterised by conversion to the picrate.

Example 9

N - Benzoyl - N′ - [3 - (4 - imidazolyl)propyl] - N″ - [2 - (5 - methyl - 4 - imidazolylmethylthio)ethyl]guanidine (2.37 g) was heated overnight on a steam bath with conc. hydrochloric acid (135 ml). The aqueous reaction mixture was evaporated to *ca* 75% of its original volume to remove the excess of hydrogen chloride. The aqueous residue obtained was extracted with diethyl ether to remove benzoic acid. The aqueous phase was evaporated at reduced pressure and azeotroped with ethanol (100 ml). The oily residue was recrystallised from ethanol to yield N - [3 - (4 - imidazolyl)propyl] - N′ - [2 - (5 - methyl - 4 - imidazolylmethylthio)ethyl]guanidine trihydrochloride (1.93 g) m.p. 200—201°C.

Example 10

A solution of 3-(5-bromo-4-imidazolyl)propylamine in ethanol was prepared *in situ* from the dihydrochloride (0.802 g) by the method of Example 1(i) with a solution of sodium ethoxide (from 0.174 g sodium and 7.5 ml ethanol) in ethanol.

The 3-(5-bromo-4-imidazolyl)propylamine solution was added dropwise with stirring over 12 minutes to a cooled (ice, 5°) solution of diphenyl benzoyliminocarbonate (1.2 g) in dichloromethane (12 ml). When the addition was complete the stirred mixture was allowed to come to room temperature over 30 minutes. The reaction mixture was concentrated at 30—40° under reduced pressure to remove solvents. The residue was dissolved in dry dimethyl formamide (6 ml) and added dropwise over 5 minutes to a stirred suspension in dry dimethylformamide (5 ml) of sodium hydride (100 mg Na H, weighed as a 50% dispersion in oil and washed before use with 40°—60° petroleum ether) cooled in ice. Stirring was continued for ca 10 minutes and the mixture was poured into water (total 100 ml). The precipitated solid from this preparation was purified by chromatography on a dry silica (25 g) column (1″ × 3″). After absorption on silica (5.0 g) from ethanol solution and evaporation. Elution with chloroform gave the required product. Concentration of the eluate gave a residue (210 mg, m.p. 166 to 168°) which was recrystallised from ethanol—water (ca 8 ml) to give, a cooling 3-bromo-8-benzamide-4,5-dihydroimidazo-(3,4-c)-1,3-diazepine (200 mg) m.p. 166 to 167°. (Found: C, 50.62;

H, 4.10; N, 16.62; Br, 23.81%. $C_{14}H_{13}BrN_{4}O$ requires: C, 50.47; H, 3.93; N, 16.82; Br, 23.98%).

Example 11

(i) A solution of 2-(5-bromo-4-imidazolyl)ethyl amine in ethanol was prepared *in situ* from the dihydrobromide (3.0 g) by the method of Example 1(i) with a solution of sodium ethoxide in ethanol (from 0.174 g sodium and 50 ml ethanol).

The 2-(5-bromo-4-imidazolyl)ethyl amine solution was added dropwise over 10 min to a cooled (5°C) solution of diphenylcyanoiminocarbonate (2.83 g) in dichloromethane (50 ml), maintaining the temperature between 5—7°C. When the addition was complete the mixture was stirred for a further 30 min at room temperature.

The solvents were removed at reduced pressure and the residue so obtained was recrystallised from methanol to yield 2-bromo-7-cyanoamino-3,4-dihydroimidazo-(3,4-c)-1,3-pyrimidine (1.6 g) as a buff coloured crystals m.p. 270—271°C.

(ii) A solution of 2-bromo-7-cyanoamino-3,4-dihydroimidazo-(3,4-c)-1,3-pyrimidine (1.2 g) and 2-(5-methyl-4-imidazolylmethylthio)ethylamine (1.49 g) in ethanol (50 ml) was refluxed for ca 20 hr with stirring. The reaction mixture was evaporated at reduced pressure to yield a residue which on trituration with acetonitrile gave a white solid. This solid was recrystallised from ethanol/acetonitrile to give N - cyano - N′ - [2 - (5 - bromo - 4 - imidazolyl)ethyl] - N″ - [2 - (5 - methyl - 4 - imidazolylmethylthio)ethyl]guanidine (1.6 g) m.p. 149—150°C.

Example 12

A solution of 4-imidazolylpropylamine in ethanol was prepared *in situ* from the dihydrochloride (2.97 g) by the method of Example 1(i) with a solution of sodium ethoxide in ethanol (from 0.69 g sodium and 50 ml ethanol).

To this was added 2 - methyl - 7 - cyano - 3,4 - dihydroimidazo(3,4-c) - 1,3 - pyrimidine (2.37 g) and the mixture was refluxed for 20 hr. The reaction mixture was evaporated and the residue chromatographed on a silica gel column, eluting with dichloromethane/methanol 5:1. Evaporation of the eluate gave a white solid which was recrystallised from water yielding N - cyano - N′ - [2 - (5 - methyl - 4 - imidazolyl)ethyl] - N″ - [3 - (4 - imidazolyl)propyl]guanidine (1.25 g) m.p. 81—82°C.

## Claims

1. A process for preparing a compound of formula (IX):—

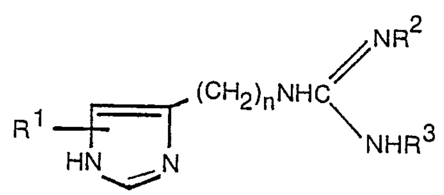

(IX)

where $R^1$ is attached to a carbon atom in the imidazole moiety and is hydrogen, halogen or alkyl; $R^2$ is hydrogen, or a group convertible into hydrogen under acid conditions; $R^3$ is an optionally substituted alkyl group and *n* is 2, 3 or 4; which comprises reacting a compound of formula (X):—

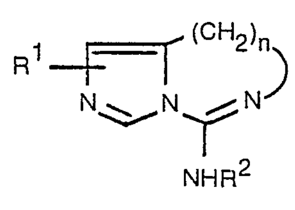

(X)

where *n* and $R^1$ are as defined with reference to formula (IX), and $R^2$ is a group convertible into hydrogen under acid conditions with a compound of formula (XI):—

$$R^3NH_2 \qquad (XI)$$

where $R^3$ is as defined with reference to formula (IX) in the presence of a polar organic solvent and thereafter optionally converting in the compound so obtained the group $R^2$ into hydrogen under acid conditions.

2. A process as claimed in claim 1 where $R^1$ is hydrogen.

3. A process as claimed in claim 1 or 2 where $R^2$ in the compound of formula (X) is benzoyl.

4. A process as claimed in any one of claims 1 to 3 where $R^3$ in the compound of formula (XI) is 2-(5-methyl-4-imidazolylmethylthio)ethyl.

5. A process as claimed in claim 1 for preparing N - [3 - (4 - imidazolyl)propyl] - N' - [2 - (5 - methylimidazolylmethylthio)ethyl]guanidine which comprises reacting 8 - benzamido - 4,5 - dihydroimidazo(3,4-c) - 1,3 - diazepine with 2-(5-methylimidazolylmethylthio)ethylamine and thereafter hydrolyzing the benzoyl group into hydrogen under acid conditions.

6. A compound of formula (X) in claim 1 where *n*, $R^1$ and $R^2$ are as defined in any one of claims 1 to 4.

7. 8-Benzamido-4,5-dihydroimidazo(3,4-c)-1,3-diazepine.

8. 9-Benzamido-4,5,6,7-tetrahydroimidazo(3,4-c)-1,3-diazocine.

9. A process for preparing a compound as claimed in any one of claims 6 to 8 which comprises cyclising in a polar organic solvent a compound of formula (XIV):—

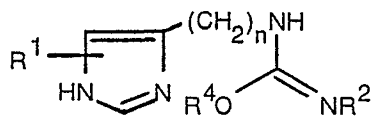

(XIV)

where $R^1$, $R^2$ and *n* are as defined with reference to formula (X) and $R^4$ is an optionally substituted aryl group; where necessary in the presence of a non-nucleophilic base.

10. A process as claimed in claim 9 where $R^4$ is phenyl.

11. A process as claimed in claim 9 or claim 10 where $R^2$ is benzoyl.

12. A process as claimed in claim 11 where the base is sodium hydride.

13. A process for preparing a compound of formula (X) in claim 1 where *n* is 2 or 3 and $R^2$ is cyano which comprises reacting in a polar organic solvent an amine of formula (XV):—

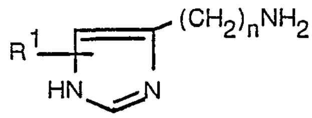

(XV)

where $R^1$ is as defined with reference to formula (XIV) in claim 9 and *n* is 2 or 3, with a compound of formula (XVI):—

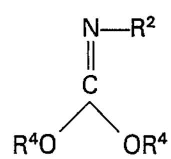

(XVI)

where $R^2$ is cyano and $R^4$ is as defined with reference to formula (XIV) to form a compound of formula (XIV) where $R^1$ is as previously defined, $R^2$ is CN and *n* is 2 or 3 which is allowed to cyclise *in situ*.

**Revendications**

1. Procédé de préparation d'un composé de formule (IX):

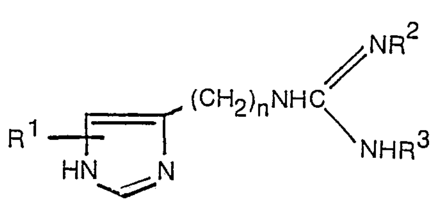

(IX)

dans laquelle $R^1$ est fixé à un atome de carbone dans le fragment imidazole et représente de l'hydrogène, un halogène ou un groupe alcoyle; $R^2$ représente de l'hydrogène ou un groupe convertible en hydrogène dans des conditions acides; $R^3$ représente un groupe alcoyle éventuellement substitué et *n* est égal à 2, 3 ou 4; qui comprend la mise en réaction d'un composé de formule (X):

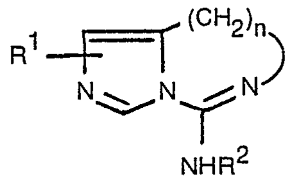

(X)

dans laquelle *n* et $R^1$ sont tels que définis à propos de la formule (IX) et $R^2$ représente un groupe convertible en hydrogène dans des conditions acides, avec un composé de formule (XI):

$R^3NH_2$ (XI)

dans laquelle $R^3$ est tel que défini à propos de la formule (IX), en présence d'un solvant organique polaire et la transformation ultérieure éventuelle dans le composé ainsi obtenu du groupe $R^2$ en hydrogène dans des conditions acides.

2. Procédé tel que revendiqué dans la revendication 1, dans lequel $R^1$ représente de l'hydrogène.

3. Procédé tel que revendiqué dans la revendication 1 ou 2, dans lequel $R^2$ représente un groupe benzoyle dans le composé de formule (X).

4. Procédé tel que revendiqué dans l'une quelconque des revendications 1 à 3, dans lequel $R^3$ représente un groupe 2-(5-méthyl-4-imidazolylméthylthio)éthyle dans le composé de formule (XI).

5. Procédé de préparation de la N - [3 - (4 - imidazolyl) - propyl) - N' - [2 - (5 - méthylimidazolylméthylthio)éthyl]guanidine, tel que revendiqué dans la revendication 1, qui comprend la mise en réaction de la 8-benzamido-4,5-dihydroimidazo[3,4-c]-1,3-diazépine avec la 2-(5-méthylimidazolylméthylthio)éthylamine et l'hydrolyse subséquente du groupe benzoyle en hydrogène dans des conditions acides.

6. Composé de formule (X) dans la revendication 1, dans laquelle *n*, $R^1$ et $R^2$ sont tels que définis dans l'une quelconque des revendications 1 à 4.

7. La 8-benzamido-4,5-dihydroimidazo[3,4-c]-1,3-diazépine.

8. La 9-benzamido-4,5,6,7-tétrahydroimidazo[3,4-c]-1,3-diazocine.

9. Procédé de préparation d'un composé tel que revendiqué dans l'une quelconque des revendications 6 à 8, qui comprend la cyclisation dans un solvant organique polaire d'un composé de formule (XIV):

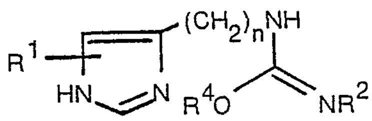

(XIV)

dans laquelle $R^1$, $R^2$ et *n* sont tels que définis à propos de la formule (X) et $R^4$ représente un groupe aryle éventuellement substitué en présence d'une base non nucléophile là où il y a lieu.

10. Procédé tel que revendiqué dans la revendication 9, dans lequel $R^4$ représente un groupe phényle.

11. Procédé tel que revendiqué dans la revendication 9 ou la revendication 10, dans lequel $R^2$ représente un groupe benzoyle.

12. Procédé tel que revendiqué dans la revendication 11, dans lequel la base est l'hydrure de sodium.

13. Procédé de préparation d'un composé de formule (X) dans la revendication 1, dans laquelle *n* est égal à 2 ou 3 et $R^2$ représente un groupe cyano qui comprend la mise en réaction dans un solvant organique polaire d'une amine de formule (XV):

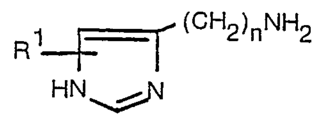

(XV)

dans laquelle $R^1$ est tel que défini à propos de la formule XIV dans la revendication 9 et *n* est égal à 2 ou 3, avec un composé de formule (XVI):

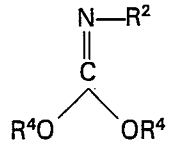

(XVI)

dans laquelle $R^2$ représente un groupe cyano et $R^4$ est tel que défini à propos de la formule (XIV) pour former un composé de formule (XIV) dans laquelle $R^1$ est tel que défini précédemment, $R^2$ représente un groupe cyano et *n* est égal à 2 ou 3, qu'on laisse cycliser *in situ*.

**Patentansprüche**

1. Verfahren zur Herstellung einer Verbindung der Formel (IX):

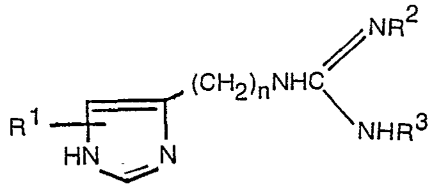

(IX)

wobei $R^1$ an ein Kohlenstoffatom des Imidazolringes gebunden ist und ein Wasserstoff- oder Halogenatom oder einen Alkylrest, $R^2$ ein Wasserstoffatom oder eine Gruppe, die sich unter sauren Bedingungen in ein Wasserstoffatom überführen läßt, und $R^3$ einen gegebenenfalls substituierten Alkylrest bedeuten und n den Wert 2, 3 oder 4 hat, dadurch gekennzeichnet, daß man eine Verbindung der Formel (X)

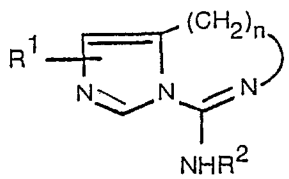

(X)

in der n und $R^1$ die angegebene Bedeutung haben und $R^2$ eine Gruppe ist, die unter sauren Bedingungen in ein Wasserstoffatom überführt werden kann, mit einer Verbindung der Formel (XI)

$$R^3NH_2 \qquad (XI)$$

in der $R^3$ die vorstehend angegebene Bedeutung hat, in Gegenwart eines polaren organischen Lösungsmittels umsetzt und danach gegebenenfalls die Gruppe $R^2$ der so erhaltenen Verbindung unter sauren Bedingungen in ein Wasserstoffatom überführt.

2. Verfahren nach Anspruch 1, dadurch gekennzeichnet, daß $R^1$ ein Wasserstoffatom ist.

3. Verfahren nach Anspruch 1 oder 2, dadurch gekennzeichnet, daß $R^2$ in der Verbindung der Formel (X) eine Benzoylgruppe ist.

4. Verfahren nach einem der Ansprüche 1 bis 3, dadurch gekennzeichnet, daß $R^3$ in der Verbindung der Formel (XI) eine 2-(5-Methyl-4-imidazolylmethylthio)-äthyl-Gruppe ist.

5. Verfahren nach Anspruch 1 zur Herstellung von N - [3 - (4 - Imidazolyl)propyl] - N' - [2 - (5 - methylimidazolylmethylthio) - äthyl] - guanidin, dadurch gekennzeichnet, daß man 8-Benzamido-4,5-dihydroimidazo-(3,4-c)-1,3-diazepin mit 2-(5-Methylimidazolylmethylthio)-äthylamin umsetzt und die Benzoylgruppe anschließend unter sauren Bedingungen durch Hydrolyse in ein Wasserstoffatom überführt.

6. Verbindung nach Anspruch 1 mit der Formel (X), dadurch gekennzeichnet, daß n, $R^1$ und $R^2$ die in den Ansprüchen 1 bis 4 angegebene Bedeutung haben.

7. 8-Benzamido-4,5-dihydroimidazo-(3,4-c)-1,3-diazepin.

8. 9-Benzamido-4,5,6,7-tetrahydroimidazo-(3,4-c)-1,3-diazocin.

9. Verfahren zur Herstellung einer Verbindung gemäß den Ansprüchen 6 bis 8, dadurch gekennzeichnet, daß man eine Verbindung der Formel (XIV)

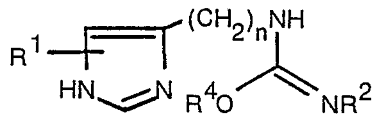

(XIV)

in der $R^1$, $R^2$ und n die vorstehend bei der Formel (X) angegebene Bedeutung haben und $R^4$ ein gegebenenfalls substituierter Arylrest ist, in einem polaren organischen Lösungsmittel und gegebenenfalls in Gegenwart einer nichtnukleophilen Base cyclisiert.

10. Verfahren nach Anspruch 9, dadurch gekennzeichnet, daß $R^4$ eine Phenylgruppe ist.

11. Verfahren nach Anspruch 9 oder 10, dadurch gekennzeichnet, daß $R^2$ eine Benzoylgruppe ist.

12. Verfahren nach Anspruch 11, dadurch gekennzeichnet, daß die Base Natriumhydrid ist.

13. Verfahren zur Herstellung der Verbindung der Formel (X) gemäß Anspruch 1, wobei n den Wert 2 oder 3 hat und $R^2$ eine Cyanogruppe ist, dadurch gekennzeichnet, daß man ein Amin der Formel (XV)

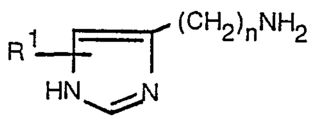

(XV)

in der $R^1$ die vorstehend bei der Formel (XIV) in Anspruch 9 angegebene Bedeutung hat und n den Wert 2 oder 3 hat, mit einer Verbindung der Formel (XVI)

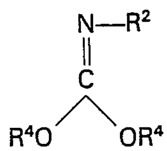

(XVI)

in der $R^2$ eine Cyanogruppe ist und $R^4$ die vorstehend bei der Formel (XIV) angegebene bedeutung hat, in einem polaren organischen Lösungsmittel umsetzt und die entstandene Verbindung der Formel (XIV) in der $R^1$ die vorstehend angegebene Bedeutung hat und $R^2$ eine Cyanogruppe ist und n der Wert 2 oder 3 hat, in situ cyclisiert.